(19) Europäisches Patentamt
European Patent Office
Office européen des brevets

(11) Veröffentlichungsnummer: **0 124 011**
**B1**

(12) # EUROPÄISCHE PATENTSCHRIFT

(45) Veröffentlichungstag der Patentschrift: **08.11.89**

(51) Int. Cl.[4]: **C 07 D 303/08**, C 07 D 303/04, C 07 D 303/22, C 07 D 301/02 // C07D249/08

(21) Anmeldenummer: **84104330.0**

(22) Anmeldetag: **17.04.84**

(54) **Verfahren zur Herstellung von Oxiranen.**

(30) Priorität: **29.04.83 DE 3315619**

(43) Veröffentlichungstag der Anmeldung: **07.11.84 Patentblatt 84/45**

(45) Bekanntmachung des Hinweises auf die Patenterteilung: **08.11.89 Patentblatt 89/45**

(84) Benannte Vertragsstaaten: **BE CH DE FR GB LI**

(56) Entgegenhaltungen:
**EP-A- 0 040 345**
**EP-A- 0 094 726**

**JOURNAL OF THE AMERICAN CHEMICAL SOCIETY, Band 95, Nr. 16, 8. August 1973, Seiten 5311-5321, Columbus, Ohio, US; B.M. TROST et al.: "New synthetic reactions. Facile synthesis of oxaspiropentanes, versatile synthetic intermediates"**

**Die Akte enthält technische Angaben, die nach dem Eingang der Anmeldung eingereicht wurden und die nicht in dieser Patentschrift enthalten sind.**

(73) Patentinhaber: **BAYER AG, D-5090 Leverkusen 1 Bayerwerk (DE)**

(72) Erfinder: **Zerbes, Rudolf, Dr., In der Beek 26, D-5600 Wuppertal 1 (DE)**
Erfinder: **Linke, Siegfried, Dr. Bayer Pharma Korea Ltd., Daehan Building 10th Floor 75, Seosomondong Choong-ku Seoul (KR)**
Erfinder: **Mohrmann, Karl Heinrich, Dr., Roonstrasse 61, D-5600 Wuppertal 1 (DE)**
Erfinder: **Reiser, Wolf, Dr., Kiebitzweg 12a, D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 124 011 B1

ACTORUM AG

**Beschreibung**

Die vorliegende Erfindung betrifft ein neues Verfahren zur Herstellung von bekannten Oxiranen, die als Zwischenprodukte zur Synthese von Verbindungen mit pflanzenwuchsregulierender und fungizider Wirksamkeit verwendet werden können.

Es ist bereits bekannt geworden, daß man Oxirane herstellen kann, indem man Dimethylsulfid und Dimethylsulfat umsetzt und das dabei intermediär entstehende Trimethylsulfonium-methylsulfat anschließend mit Carbonyl-Verbindungen in Gegenwart eines inerten organischen Lösungsmittels und in Gegenwart einer starken Base, wie Butyl-lithium, Natriumhydrid, Natriumamid, Kalium-tert.-butylat, Natriummethylat oder Natriumethylat zur Reaktion bringt (vgl. J. Amer. Chem. Soc. 87, 1353–1364 (1965) und Ber. 96, 1881 (1963)). So läßt sich zum Beispiel 2-(4-Chlorphenoxymethyl)-2-tert.-butyloxiran herstellen, indem man aus Dimethylsulfid und Dimethylsulfat zubereitetes Trimethylsulfonium-methylsulfat in situ mit 1-(4-Chlorphenoxy)-3,3-dimethyl-butan-2-on in Acetonitril in Gegenwart von Natriummethylat umsetzt (vgl. EP-A-40 345). Die Ausbeuten bei diesem Verfahren sind gut. Nachteilig ist jedoch, daß die verwendbaren Basen jeweils speziell hergestellt werden müssen und schwierig zu handhaben sind, da sie feuchtigkeitsempfindlich und zum Teil auch brennbar sind.

Weiterhin ist bekannt, daß man Oxirane erhält, wenn man Dimethylsulfid mit Dimethylsulfat behandelt und das dabei anfallende Trimethylsulfonium-methylsulfat mit Carbonyl-Verbindungen in Gegenwart von konzentrierter wäßriger Natronlauge, einem mit Wasser wenig mischbaren organischen Lösungsmittel sowie in Gegenwart eines Phasen-Transfer-Katalysators umsetzt (vgl. Angew. Chem. 85, 867–868 (1973) und J. Org. Chem. 34, 2133 (1969)). Nach diesem Verfahren wurden bisher allerdings nur Aldehyde in Oxirane überführt. Außerdem ist in dem aus zwei flüssigen Phasen bestehenden System jeweils die Anwesenheit eines Phasen-Transfer-Katalysators erforderlich.

Schließlich geht aus der Veröffentlichung in Tetrahedron Letters 23, 5283–5286 (1982) hervor, daß festes Kaliumhydroxid eingesetzt werden kann, um Aldehyde mit Hilfe von Sulfonium-Salzen in Ausbeuten von mehr als 90% in die entsprechenden Oxirane umzuwandeln. Dagegen entstehen aus Ketonen die zugehörigen Oxirane bei dieser Herstellungsmethode nur in sehr bescheidenen Ausbeuten von 28% bzw. 38% (vgl. zweite und dritte Zeile von unten in der Tabelle).

Es wurde nun gefunden, daß man die bekannten Oxirane der Formel

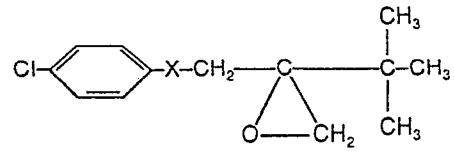 (I)

in welcher

X für Sauerstoff oder $CH_2$ steht,

erhält, wenn man Dimethylsulfid mit Dimethylsulfat in Gegenwart eines inerten organischen Verdünnungsmittels behandelt und das dabei entstehende Trimethylsulfoniummethylsulfat der Formel

$$(CH_3)_3S^{\oplus}\ CH_3SO_4^{\ominus} \qquad (II)$$

ohne vorherige Isolierung mit einem Keton der Formel

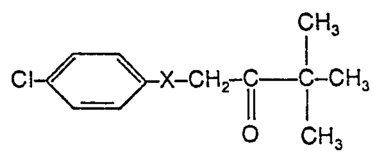 (II)

in welcher

X die oben angegebene Bedeutung hat,

in Gegenwart von Kaliumhydroxid- bzw. Natriumhydroxid-Pulver sowie in Gegenwart eines inerten organischen Verdünnungsmittels bei Temperaturen zwischen 0 °C und 60 °C umsetzt.

Es ist als äußerst überraschend zu bezeichnen, daß sich Oxirane der Formel (I) nach dem erfindungsgemäßen Verfahren in höherer Ausbeute herstellen lassen als nach dem bisher bekannten Verfahren. Im übrigen war aufgrund des bekannten Standes der Technik anzunehmen, daß bei einer derartigen Umsetzung von Ketonen, die ja im allgemeinen weniger reaktiv sind als entsprechende Aldehyde, die Anwesenheit eines Phasen-Transfer-Katalysators erforderlich wäre. Im Gegensatz zu den Erwartungen ist das jedoch nicht nötig. Die Umsetzung verläuft auch in Abwesenheit eines Katalysators problemlos, obwohl sie in einem System aus einer festen und einer flüssigen Phase stattfindet. Überraschend ist außerdem, daß sich die erfindungsgemäße Umsetzung bei Verwendung von Natriumhydroxid oder Kaliumhydroxid ohne Schwierigkeiten durchführen läßt, während die gleiche Umsetzung bei Verwendung von pulverisiertem Lithiumhydroxid als Base unbefriedigende Ergebnisse liefert.

Das erfindungsgemäße Verfahren zeichnet sich durch eine Reihe von Vorteilen aus. So sind die einsetzbaren Basen auch in technischem Maßstab verfügbar, einfach zu handhaben und nicht brennbar. Ferner sind die Oxirane der Formel (I) auf diese Weise in sehr hohen Ausbeuten zugänglich.

Verwendet man bei dem erfindungsgemäßen Verfahren 1-(4-Chlor-phenoxy)-3,3-dimethyl-butan-2-on als Ausgangsstoff und Kaliumhydroxid-Pulver als Base, so kann der Reaktionsablauf durch das folgende Formelschema veranschaulicht werden:

a) $(CH_3)_2S + (CH_3)_2SO_4 \longrightarrow (CH_3)_3S^{\oplus}CH_3SO_4^{\ominus}$

b) 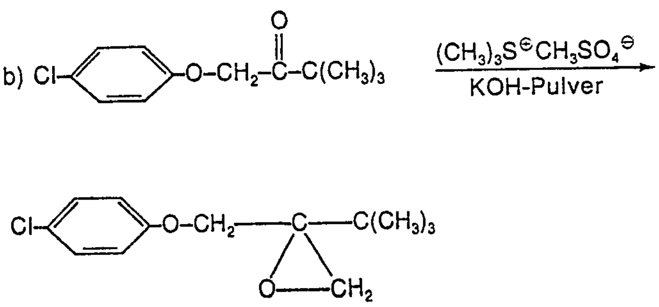


Die Ketone der Formel (III) sind bekannt (vgl. DE-C-2 201 063, DE-A-2 705 678 und DE-A-2 737 489).

Das bei dem erfindungsgemäßen Verfahren weiterhin als Ausgangsstoff benötigte Trimethylsulfonium-methylsulfat der Formel (II) ist ebenfalls bekannt (vgl. Heterocycles 8, 397 (1977)). Es wird bei der obigen Umsetzung in frisch hergestelltem Zustand eingesetzt, indem man es durch Reaktion von Dimethylsulfid mit Dimethylsulfat in situ erzeugt.

Als Basen dienen bei dem erfindungsgemäßen Verfahren pulverisiertes Kaliumhydroxid oder pulverisiertes Natriumhydroxid.

Als Verdünnungsmittel können bei dem erfindungsgemäßen Verfahren sowohl bei der Herstellung des Trimethylsulfonium-methylsulfats als auch bei der anschließenden Umsetzung des Stoffes mit einem Keton der Formel (III) alle inerten organischen Lösungsmittel verwendet werden. Vorzugsweise in Frage kommen Nitrile, wie Acetonitril, ferner polare Solventien, wie Dimethylsulfoxid, außerdem aliphatische oder aromatische Kohlenwasserstoffe, wie Hexan, Benzol, Toluol oder Xylol, und schließlich Alkohole, wie tert.-Butanol und Isopropanol, und darüber hinaus auch N-Methyl-pyrrolidon.

Die Reaktionstemperaturen können bei der Durchführung des erfindungsgemäßen Verfahrens innerhalb eines bestimmten Bereiches variiert werden. Bei der Herstellung des Trimethylsulfoniummethylsulfats als auch bei dessen anschließender Umsetzung mit einem Keton der Formel (III) soll man bei Temperaturen zwischen 0 °C und 60 °C, vorzugsweise zwischen 10 °C und 40 °C arbeiten.

Das erfindungsgemäße Verfahren wird im allgemeinen bei Normaldruck durchgeführt. Es ist aber auch möglich, bei erhöhtem oder vermindertem Druck zu arbeiten.

Bei der Durchführung des erfindungsgemäßen Verfahrens werden die Mengen an den Reaktionskomponenten im allgemeinen so gewählt, daß auf 1 Mol an Keton der Formel (III) 1,0 bis 2,0 Mol, vorzugsweise 1,0 bis 1,5 Mol an Dimethylsulfat, 1,0 bis 2,2 Mol, vorzugsweise 1,0 bis 1,6 Mol an Dimethylsulfid und 1,0 bis 4,0 Mol, vorzugsweise 1,5 bis 2,0 Mol an Kaliumhydroxid bzw. Natriumhydroxid eingesetzt werden.

Im einzelnen verfährt man bei dem erfindungsgemäßen Verfahren in der Weise, daß man Dimethylsulfid und Dimethylsulfat in einem Lösungsmittel zusammengibt, diese Lösung dann mit einer Lösung von Keton der Formel (III) in einem organischen Solvens versetzt und anschließend die jeweils gewünschte Menge an Base hinzufügt. Die Aufarbeitung erfolgt nach üblichen Methoden. Im allgemeinen geht man so vor, daß man zunächst das Lösungsmittel abzieht, den verbleibenden Rest mit Oxidationsmitteln, wie wäßriger Wasserstoffperoxid-Lösung, verdünnter wäßriger Natrium- oder Kalium-hypochlorit-Lösung, oder auch mit einem Gemisch aus Wasser und einem mit Wasser wenig mischbaren organischen Lösungsmittel versetzt, die organische Phase abtrennt, wäscht und nach gegebenenfalls vorherigem Trocknen einengt. Das dabei anfallende Produkt kann zur weiteren Reinigung unter vermindertem Druck destilliert werden.

Die nach dem erfindungsgemäßen Verfahren herstellbaren Oxirane der Formel (I) sind wertvolle Ausgangsstoffe zur Synthese von 1-Hydroxyethyl-azol-Derivaten, welche hervorragende pflanzenwuchsregulierende und fungizide Eigenschaften besitzen (vgl. EP-A-40 345).

So läßt sich beispielsweise das 2-(4-Chlor-phenoxymethyl)-3,3-dimethyl-1-(1,2,4-triazol-1-yl)-butan-2-ol der Formel

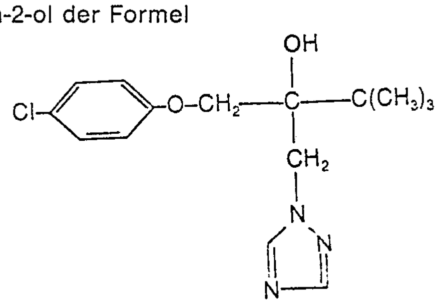

herstellen, indem man 2-(4-Chlor-phenoxymethyl)-2-tert.-butyl-oxiran mit 1,2,4-Triazol in Gegenwart von Kaliumhydroxid umsetzt. Diese Synthese läßt sich formelmäßig wie folgt veranschaulichen:

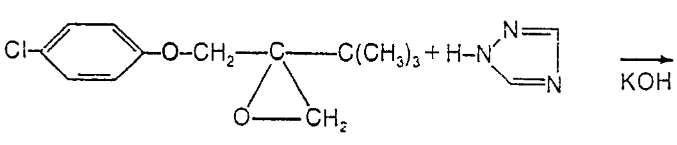

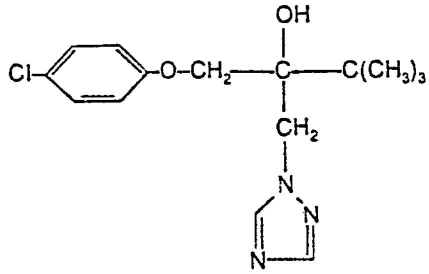

(I)

Das erfindungsgemäße Verfahren wird durch die nachfolgenden Beispiele veranschaulicht.

Beispiel 1

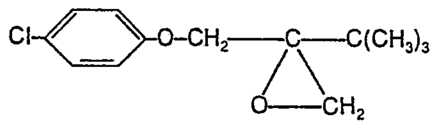

In 400 ml Acetonitril wurden bei Raumtemperatur 27 ml (0,375 Mol) Dimethylsulfid und 30 ml (0,317 Mol) Dimethylsulfat gegeben. Diese Mischung wurde zur Bildung des Trimethylsulfonium-methylsulfats 12 Stunden bei Raumtemperatur stehen gelassen und dann unter Rühren tropfenweise mit einer Lösung von 57 g (0,25 Mol) 1-(4-Chlorphenoxy)-3,3-dimethyl-butan-2-on in 100 ml Toluol versetzt. Danach wurden unter äußerer Kühlung mit Eiswasser und unter Rühren 25,8 g (0,46 Mol) gemahlenes Kaliumhydroxid portionsweise hinzugefügt. Man rührte weitere 12 Stunden bei Raumtemperatur und destillierte dann 400 ml Lösungsmittel ab. Das verbleibende Gemisch wurde abgekühlt, mit 300 ml einer verdünnten wäßrigen Natriumhypochlorit-Lösung versetzt und 5 Minuten gerührt. Die organische Phase wurde abgetrennt, zweimal mit je 100 ml Wasser gewaschen und dann unter vermindertem Druck eingedampft. Es verblieb ein Rückstand von 61,4 g, der gemäß Gaschromatogramm zu 93,6% aus 2-(4-Chlor-phenoxy-methyl)-2-tert.-butyl-oxiran bestand. Danach errechnet sich eine Ausbeute von 95,6% der Theorie.

Beispiel 2

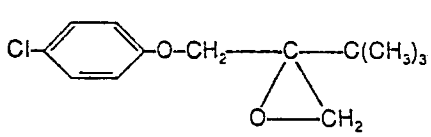

Der im Beispiel 1 beschriebene Ansatz wurde wiederholt, jedoch wurden anstelle von Kaliumhydroxid jetzt 18,4 g (0,46 Mol) gemahlenes Natriumhydroxid eingesetzt. Man erhielt nach dem Aufarbeiten 57,8 g eines Rückstandes, der gemäß Gaschromatogramm zu 90,9% aus 2-(4-Chlorphenoxy-methyl)-2-tert.-butyl-oxiran bestand. Danach errechnet sich eine Ausbeute von 87,4% der Theorie.

Vergleichsbeispiel

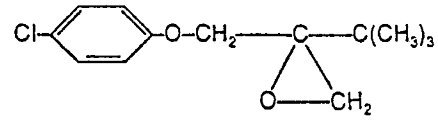

Eine Lösung von 189 ml (2,0 Mol) Dimethylsulfat in 1200 ml absolutem Acetonitril wurde bei Raumtemperatur mit einer Lösung von 162 ml (2,2 Mol) Dimethylsulfid in 400 ml absolutem Acetonitril versetzt. Man ließ die Reaktionsmischung über Nacht bei Raumtemperatur rühren. Danach wurden 118,8 g (2,2 Mol) Natriummethylat zugegeben. Man ließ 30 Minuten rühren und versetzte dann innerhalb von 30 Minuten tropfenweise mit einer Lösung von 272 g (1,2 Mol) 1-(4-Chlorphenoxy)-3,3-dimethyl-butan-2-on in 600 ml absolutem Acetonitril.

Man ließ das Reaktionsgemisch über Nacht nachrühren. Anschließend wurde eingeengt, der Rückstand zwischen Wasser und Essigester verteilt, die organische Phase abgetrennt, zweimal mit Wasser und einmal mit gesättigter Natriumchloridlösung gewaschen, über Natriumsulfat getrocknet, eingeengt und der Rückstand im Vakuum destilliert.

Man erhielt 242,4 g (84% der Theorie) 2-(4-Chlorphenoxy-methyl)-2-tert.-butyl-oxiran vom Siedepunkt 115 bis 122 °C / 0,003 mm Hg -Säule, vom Schmelzpunkt 50 bis 52 °C.

Beispiel für die Verwendung eines erfindungsgemäß herstellbaren Oxirans zur Synthese eines 1-Hydroxyethylazol-Derivates mit pflanzenwuchsregulierender und fungizider Wirksamkeit

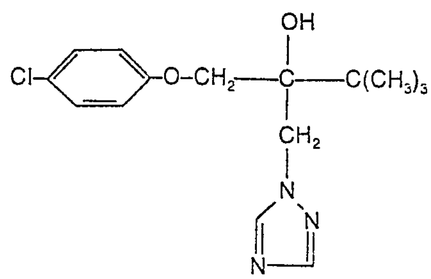

Ein Gemisch aus 30 g 2-(4-Chlor-phenoxy-methyl)-2-tert.-butyl-oxiran, 9,8 g 1,2,4-Triazol und 1,2 g festem Kaliumhydroxid in 70 ml Toluol und 7,5 ml Dimethylsulfoxid wurde 6 Stunden unter Rückfluß erhitzt. Nach dem Abkühlen auf Raumtemperatur wurde das Reaktionsgemisch mit 2 g Kieselgel versetzt und anschließend filtriert. Das Filtrat wurde mit 75 ml halbkonzentrierter Salzsäure versetzt, und die sich dabei abscheidenden Kristalle wurden abgesaugt. Danach wurde der kristalline Feststoff mit 80 ml Wasser und wäßriger Natronlauge versetzt, so daß der pH-Wert des Gemisches 13 betrug. Es wurde zwei Stunden bei Raumtemperatur nachgerührt, dann abgesaugt und mit Wasser neutral gewaschen. Man erhielt auf diese Weise 20 g (52% der Theorie) an 2-(4-Chlor-phenoxy-methyl)-3,3-dimethyl-1-(1,2,4-triazol-1-yl)-butan-2-ol
vom Schmelzpunkt 84 bis 87 °C.

**Patentansprüche**

1. Verfahren zur Herstellung von Oxiranen der Formel

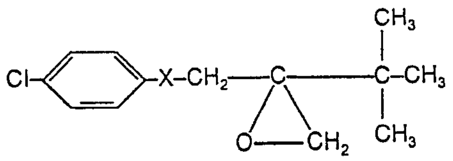
(I)

in welcher

X für Sauerstoff oder $CH_2$ steht,

dadurch gekennzeichnet, daß man Dimethylsulfid mit Dimethylsulfat in Gegenwart eines inerten organischen Verdünnungsmittels behandelt und das dabei entstehende Trimethylsulfoniummethylsulfat der Formel

$$(CH_3)_3S^{\oplus}\ CH_3SO_4^{\ominus} \quad (II)$$

ohne vorherige Isolierung mit einem Keton der Formel

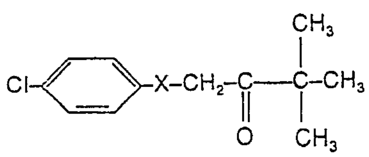
(II)

in welcher

X die oben angegebene Bedeutung hat,

in Gegenwart von Kaliumhydroxid- bzw. Natriumhydroxid-Pulver sowie in Gegenwart eines inerten organischen Verdünnungsmittels bei Temperaturen zwischen 0 °C und 60 °C umsetzt.

2. Verfahren gemäß Anspruch 1, dadurch gekennzeichnet, daß man die Umsetzung bei Temperaturen zwischen 10 °C und 40 °C durchführt.

3. Verfahren gemäß Anspruch 1, dadurch gekennzeichnet, daß man als organische Verdünnungsmittel Acetonitril, Dimethylsulfoxid, Toluol und/oder tert.-Butanol einsetzt.

4. Verfahren gemäß Anspruch 1, dadurch gekennzeichnet, daß man als Keton der Formel (III) 2-(4-Chlorphenoxy)-3,3-dimethyl-butan-2-on einsetzt.

**Claims**

1. Process for the preparation of oxiranes of the formula

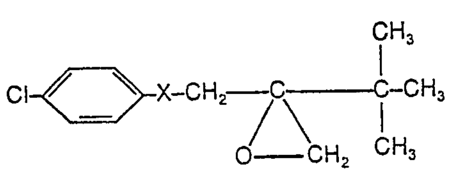
(I)

in which

X represents oxygen or $CH_2$ characterised in that dimethyl sulphide is treated with dimethyl sulphate in the presence of an inert organic diluent, and the trimethylsulphonium methyl sulphate thereby produced of the formula

$$(CH_3)_3S^{\oplus}\ CH_3SO_4^{\ominus} \quad (II)$$

is reacted, without previous isolation, with a ketone of the formula

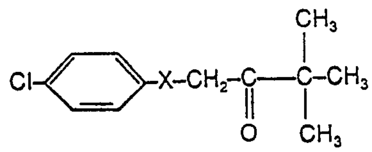
(III)

in which

X has the meaning indicated above, in the presence of potassium hydroxide or sodium hydroxide powder and in the presence of an inert organic diluent at temperatures between 0 °C and 60 °C.

2. Process according to Claim 1, characterised in that the reaction is carried out at temperatures between 10 °C and 40 °C.

3. Process according to Claim 1, characterised in that acetonitrile, dimethyl sulphoxide, toluene and/or tert.-butanol are employed as the organic diluents.

4. Process according to Claim 1, characterised in that 2-(4-chlorophenoxy)-3,3-dimethyl-2-butanone is employed as the ketone of the formula (III).

**Revendications**

1. Procédé de préparation d'oxirannes de formule

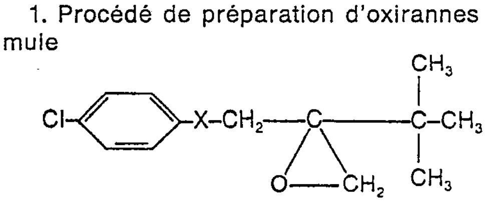
(I)

dans laquelle X représente l'oxygène ou $CH_2$, caractérisé en ce que l'on traite le sulfure de diméthyle par le sulfate de diméthyle en présence d'un diluant organique inerte, ce qui donne le méthylsulfate de triméthylsulfonium de formule

$$(CH_3)_3S^{\oplus}\ CH_3SO_4^{\ominus} \quad (II)$$

qu'on fait réagir sans l'isoler avec une cétone de formule

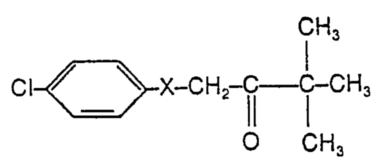
(II)

dans laquelle

X a les significations indiquées ci-dessus,

en présence d'hydroxyde de potassium ou d'hydroxyde de sodium en poudre et en présence d'un diluant organique inerte, à des températures de 0 à 60 °C.

2. Procédé selon la revendication 1, caractérisé en ce que l'on effectue la réaction à des températures de 10 à 40 °C.

3. Procédé selon la revendication 1, caractérisé en ce que l'on utilise en tant que diluants organiques l'acétonitrile, le diméthylsulfoxyde, le toluène et/ou le tert-butanol.

4. Procédé selon la revendication 1, caractérisé en ce que l'on utilise en tant que cétone de formule III la 2-(4-chlorophénoxy)-3,3-diméthyl-butane-2-one.